Europäisches Patentamt

European Patent Office

Office européen des brevets

(11) Veröffentlichungsnummer: **0 142 573**
A1

(12) # EUROPÄISCHE PATENTANMELDUNG

(21) Anmeldenummer: 83111594.4

(22) Anmeldetag: 19.11.83

(51) Int. Cl.⁴: **A 61 B 1/30**
**A 61 B 1/00**

(43) Veröffentlichungstag der Anmeldung:
29.05.85 Patentblatt 85/22

(84) Benannte Vertragsstaaten:
AT BE CH DE FR GB IT LI NL SE

(71) Anmelder: Storz, Karl
Auf dem Schildrain 39
D-7200 Tuttlingen(DE)

(72) Erfinder: Storz, Karl
Auf dem Schildrain 39
D-7200 Tuttlingen(DE)

(74) Vertreter: Wenzel, Joachim, Dipl.-Ing.
Hauptmannsreute 46
D-7000 Stuttgart 1(DE)

(54) Endoskop, insbesondere Ureteroskop, mit einem Kanal.

(57) Bei einem Endoskop (1), insbesonders Ureteroskop, mit einem Kanal zur Führung eines medizinischen Instrumentes wie z.B. einer Nadel (2) soll erreicht werden, daß eine Ablenkung der Nadel oder dargleichen bis zu 90° und eine Längsverschiebung in der abgelenkten Stellung möglich ist. Hierzu wird die Nadel in einer Innenhülse (3) des Endoskopes geführt, die ihrerseits in einer Außenhülse (4) längs verschieblich ist. Die Innenhülse zeigt an ihrem distalen Ende ein Celenk (5), das durch eine Schrägfläche (6) am distalen Ende der Außenhülse betätigbar ist. Dadurch wird die Beweglichkeit der Nadel stark erhöht, und as kann auch eine 90°–Optik Verwendung finden.

Fig. 1

EP 0 142 573 A1

<u>Karl Storz, Tuttlingen</u>

Endoskop, insbesondere Ureteroskop, mit einem Kanal

Die Erfindung bezieht sich auf ein Endoskop nach dem Oberbegriff des Anspruchs 1.

Es ist bekannt, die mit einer biegsamen Kanüle verbundene
Nadel unter radiologischer Kontrolle vom Hohlsystem der
Niere durch Parenchym und Haut hindurchzuführen. Dabei müssen gewisse Bewegungen mit einem Führungskatheter ausgeführt
werden, um die gewünschte Stelle mit der Nadel zu treffen.
Hierbei ist die radiologische Kontrolle sehr schwierig und
gelingt nicht in allen Fällen.

Dem gegenüber ist die Anwendung eines Uretereoskopes mit
einem Kanal unter endoskopischer Sichtkontrolle einfacher.
Hierbei wird das Ureteroskop durch die Harnröhre und die
Blase in das Hohlsystem der Niere eingeführt. Mit dem teleskopischen Sehrohr kann nun das Hohlsystem besichtigt werden
und die Nadel durch den Kanal unter Sichtkontrolle geführt
werden. Hierbei bestehen jedoch Schwierigkeiten, weil die
durch den Kanal des Ureteroskopes eingeführte Nadel nicht
lenkbar ist. Bei der Enge des Kanals ist ein Einbau eines
Albarran'schen Lenkhebels nicht möglich.

/-2

Es ist auch schon bekannt, biegsame Operationsinstrumente durch Schrägflächen in das Blickfeld der Optik des Endoskopes zu lenken. Diese Einrichtungen eignen sich aber nur für Endoskope mit einer geringen Ablenkung der Sehachse, weil eine steile Aufrichtung des Operationsinstrumentes beispielsweise bis zu 90° gegenüber der Längsachse des Endoskopschaftes durch Aufschieben auf eine Schrägfläche nicht möglich ist.

Der Erfindung liegt die Aufgabe zugrunde, eine Ablenkung des Operationsinstrumentes bis zu 90° und eine Längsverschiebung des Instrumentes in der abgelenkten Stellung zu ermöglichen.

Zur Lösung dieser Aufgabe sind die kennzeichnenden Merkmale des Anspruchs 1 vorgesehen. Dadurch wird die erwähnte Aufgabe gelöst, sodaß die Beweglichkeit der Nadel stark erhöht ist und auch eine 90° - Optik bei dem erwähnten Durchstoßen der Niere durch das Nierenparenchym und durch die Haut Verwendung finden kann.

Dabei besteht auch die Möglichkeit, die Auslenkstellung der Nadel oder dergleichen zu arretieren.

A-3

- 3 -

Weitere Vorteile und Einzelheiten der Erfindung ergeben
sich aus der nun folgenden Beschreibung einiger Ausführungsbeispiele unter Hinweis auf die Zeichnung. In dieser
zeigen:

Fig. 1 eine stark vergrößernde Seitenansicht auf das distale
       Ende des Endoskopes mit einem Ausschnitt;

Fig. 2 eine Seitenansicht auf die Außenhülse für sich allein;

Fig. 3 eine Draufsicht auf die Außenhülse nach Fig. 2;

Fig. 4 eine Seitenansicht auf die Innenhülse mit eingeführter Nadel;

Fig. 5 eine Seitenansicht auf die Arretiervorrichtung am
       proximalen Ende und

Fig. 6 eine schematische Seitenansicht auf ein in eine Niere
       eingeführtes Endoskop.

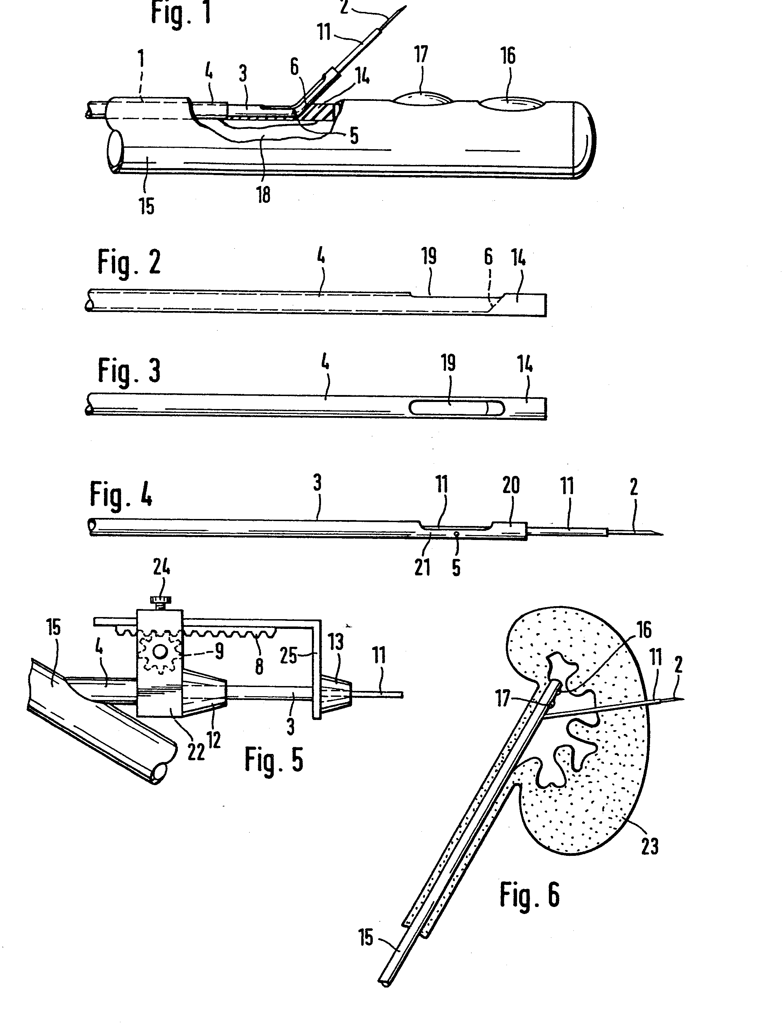

Fig. 1 zeigt nur das distale Ende des Endoskopes, dessen Schaft
15 rechts für die Lichtquelle 16 unterbrochen ist, dessen Lichtkegel etwa im rechten Winkel zur Längserstreckung des Endoskopes gerichtet ist. Weiter links sieht man ebenfalls mit einer
Blickrichtung von zum Beispiel 9o $^\circ$ das Objektiv 17 des Endoskopes, das ebenso wie die Verkleidung der Lichtquelle 16
den Außendurchmesser des Endoskopschaftes 15 nur wenig oder

/-4

- 4 -

gar nicht überragt, damit diese Teile beim Einführen in
die Körperhöhlen nicht stören. Endoskope dieser Art sind
dem Fachmann bestens bekannt und müssen deshalb nicht näher
beschrieben werden. Der Erfindungsgegenstand ist bevorzugt
bei Ureteroskopen anwendbar, die unter anderem in die Niere
einführbar sind, wie anhand der Fig. 6 noch erläutert werden
wird.

Weiter links zum distalen Ende ist ein vergrößernder Ausschnitt
18 einer weiteren seitlichen Öffnung des Endoskopschaftes 15
sichtbar. Daraus ergibt sich, daß die Lichtquelle 16, das Objektiv 17 und die seitliche Öffnung 18 in einer geraden Reihe
vom distalen zum proximalen Ende hin angeordnet sind. Das Endoskop erstreckt sich natürlich noch sehr viel weiter nach
links zum proximalen Ende, dessen Arretiervorrichtung noch
erläutert wird.

In den Kanal ist die Außenhülse 4 eingeschoben, in der die
Innenhülse 3 liegt, die ihrerseits die Nadel 2 mit der sich
daran anschließenden biegsamen Kanüle 11 aufnimmt.

Die starke Ablenkung um mehr als 45 ° der Nadel 2 ist durch
das Keilstück 14 erfolgt, weil die Innenhülse 3 mit einem
scharnierartigen Gelenk 5 versehen ist, wie man auch der
Fig. 4 entnehmen kann.

/-5

- 5 -

Die Fig. 2-4 zeigen Einzelteile des Erfindungsgegenstandes. In Fig. 2 sieht man die erwähnte Außenhülse 4 in Seitenansicht, wodurch mit unterbrochenen Linien die Schrägfläche 6 am distalen Ende innerhalb der seitlichen Öffnung 19 erkennbar ist, welche das Keilstück 14 begrenzt.

Die Draufsicht in Fig. 3 zeigt deutlich die erwähnte Öffnung 19 für den Durchgang der Innenhülse 3.

Fig. 4 zeigt nun diese Innenhülse 3, in die die Nadel 2 eingeschoben ist, an die sich die biegsame Kanüle 11 anschließt. Die Nadel 2 mit der Kanüle ragt am rechten distalen Ende 2o der Innenhülse 3 ein Stück nach rechts heraus.

Dieses Ende 2o der Innenhülse 3 ist rohrförmig ausgebildet und verjüngt sich nach links zum proximalen Ende, wobei es durch das scharnierartige Gelenk 5 mit einer entsprechenden Verjüngung 21 des weiteren Teiles des Innenhülse verbunden ist. Über dem Gelenk 5 sieht man die biegsame Kanüle 11.

Auf diese Weise läßt sich das Ende 2o der Innenhülse bis zu 9o ° nach oben um das scharnierartige Gelenk 5 schwenken.

/-6

PATENTANWALT DIPL.-ING. J. WENZEL 7 STUTTGART HAUPTMANNSREUTE 46

- 6 -

Fig. 5 zeigt eine Seitenansicht auf das proximale Ende des
Endoskopschaftes 15, in das die Außenhülse 4 seitlich in
eine Öffnung eingeschoben ist, wie dies dem Fachmann bekannt
ist. Auf der Außenhülse 4 sitzt eine Arretiervorrichtung, die
die beiden kurzen Gummihülsen 12 und 13 zum Zwecke der Dichtung aufweist. Dabei umfaßt die Gummihülse 12 gleichzeitig
die Außenhülse 4 und die Innenhülse 3 an ihrem sich verjüngenden Ende. Die Gummihülse 13 umfasst dagegen gleichzeitig die
Innenhülse 3 und die biegsame Kanüle 11 an ihrem sich verjüngenden Ende.

Am Ende der Außenhülse 4 ist eine Zahnstangenführung 22 vorgesehen, die oben die Führung für die Zahnstange 8 enthält,
in die die Schraube 24 zur Arretierung der Zahnstange eingeschraubt ist. Durch Anziehen dieser Schraube 24 von Hand kann
die Zahnstange 8 in ihrer Lage arretiert werden. Wie ersichtlich, ist die Zahnstange mit einem nach unten gerichteten Winkel 25 versehen, der mit der Innenhülse 3 fest verbunden ist.
Darüberhinaus ist in der Fig. 5 mit unterbrochenen Linien ein
Zahnrad 9 sichtbar, das in die Zahnstange 8 eingreift. Durch
Verstellen des Zahnrades 9 von Hand kann bei gelöster Feststellschraube 24 die Innenhülse 3 gegenüber der Außenhülse 4
um einen definierten Abstand verschoben werden.

PATENTANWALT DIPL.-ING. J. WENZEL 7 STUTTGART HAUPTMANNSREUTE 46

- 7 -

Fig. 6 zeigt schematisch den Innenraum einer Niere 23,
in die das verkürzt dargestellte Endoskop 15 eingeführt
ist. Man sieht auch bereits die Nadel 2, die in einem Winkel größer als 45 ° unterBeobachtung durch das Objektiv 17
und Bestrahlung durch die Lichtquelle 16 durch die Niere
23 hindurchgestoßen worden ist.

Zum Einführen des Endoskopes ruht die Nadel 2 vollständig
in dem Kanal 1 und steht nicht über diesen hinaus. Erst
dann,wenn durch die Beobachtung die zu durchstoßende Stelle
gefunden ist, wird durch eine Längsverschiebung der Innenhülse 3 gegenüber der Außenhülse 4 gemäß Fig. 5 die Nadel
herausgeführt und in dem benötigten Winkel eingestellt.
Erst danach erfolgt dann die geradlinige Bewegung der Nadel
durch eine Verschiebung der bigsamen Kanüle 11 gegenüber der
Innenhülse 3. Erst damit wird die Niere schließlich durchstoßen.

Die Erfindung ist nicht auf die dargestellten Ausführungsformen beschränkt. Vielmehr können in andere Körperhöhlen
andere Sonden, Zangen, Katheter und dergleichen Instrumente
eingeführt werden.

PATENTANWALT DIPL.-ING. J. WENZEL 7 STUTTGART HAUPTMANNSREUTE 46

## Ansprüche

1. Endoskop, insbesondere Ureteroskop, mit einem Kanal (1) zur Führung eines medizinischen Instrumentes wie zum Beispiel einer Nadel (2), dadurch gekennzeichnet, daß die Nadel oder dergleichen in einer Innenhülse (3) geführt ist, die ihrerseits in einer Außenhülse (4) längsverschieblich ist, wobei die Innenhülse an ihrem distalen Ende ein Gelenk (5) aufweist, das durch eine Schrägfläche (6) am distalen Ende der Außenhülse betätigbar ist.

2. Endoskop nach Anspruch 1, dadurch gekennzeichnet, daß die Innenhülse (3) an der Außenhülse (4) in einer verstellbaren Lage durch eine Arretiervorrichtung (8,9,22) am proximalen Ende des Endoskopes feststellbar und /oder bewegbar ist.

3. Endoskop nach Anspruch 2, dadurch gekennzeichnet, daß die Arretiervorrichtung ein Zahnstangengetriebe (8,9) aufweist.

0142573

Fig. 1

Fig. 2

Fig. 3

Fig. 4

Fig. 5

Fig. 6

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-1 767 025  (R.H. WAPPLER) * Seite 1, Zeile 97 - Seite 3, Zeile 32; Figuren 1-6 * | 1 | A 61 B    1/30 A 61 B    1/00 |
| A | FR-A- 636 912  (A. SASS & R. WOLF) * Insgesamt * | 1 | |
| A | DE-A-2 351 451  (OLYMPUS OPTICAL CO., LTD.) * Seite 2, Zeile 1 - Seite 5, Zeile 5; Figuren 1,3 * | 1 | |
| A | US-A-1 453 975  (G. GREENBERG & W.S. WINTER) * Seite 3, Zeilen 55-127; Figuren 9-16 * | 1-3 | |
| A | WO-A-8 002 499  (AMERICAN CYSTOSCOPES MAKERS) * Seite 6, Zeile 27 - Seite 11, Zeile 24; Figuren 1-10 * | 1,2 | RECHERCHIERTE SACHGEBIETE (Int. Cl. ³) A 61 B    1 A 61 B    17 |
| A | US-A-2 038 394  (F.C. WAPPLER) * Insgesamt * | 1-3 | |

Der vorliegende Recherchenbericht wurde für alle Patentansprüche erstellt.

| Recherchenort | Abschlußdatum der Recherche | Prüfer |
|---|---|---|
| DEN HAAG | 11-07-1984 | ZILLIOX J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503. 03.82